(19) Europäisches Patentamt
European Patent Office
Office européen des brevets

(11) EP 0 306 597 B2

(12) NEW EUROPEAN PATENT SPECIFICATION

(45) Date of publication and mention of the opposition decision:
**16.10.2002 Bulletin 2002/42**

(45) Mention of the grant of the patent:
**02.11.1994 Bulletin 1994/44**

(21) Application number: **88101795.8**

(22) Date of filing: **14.03.1986**

(51) Int Cl.[7]: **C07H 19/073**, C07H 19/10, A61K 31/70

# (54) Antiviral nucleosides

Antivirale Nukleoside

Nucléosides antiviraux

(84) Designated Contracting States:
**AT BE CH DE FR GB IT LI LU NL SE**

(30) Priority: **16.03.1985 GB 8506869**
**09.05.1985 GB 8511774**
**27.09.1985 GB 8523881**
**12.02.1986 GB 8603450**
**17.09.1985 US 776899**

(43) Date of publication of application:
**15.03.1989 Bulletin 1989/11**

(62) Document number(s) of the earlier application(s) in accordance with Art. 76 EPC:
**86301897.4 / 0 196 185**

(73) Proprietor: **THE WELLCOME FOUNDATION LIMITED**
**Greenford, Middlesex UB6 0NN (GB)**

(72) Inventors:
- **Rideout, Janet Litster**
  **Raleigh North Carolina (US)**
- **Barry, David Walter**
  **Chapel Hill North Carolina (US)**
- **Lehrman, Sandra Nusinoff**
  **Durham North Carolina (US)**
- **St Clair, Martha Heider**
  **Durham North Carolina (US)**
- **Furman, Phillip Allen**
  **Durham North Carolina (US)**
- **Freeman, George Andrew**
  **Cary North Carolina (US)**

(74) Representative: **Blakey, Alison Jane et al**
**GlaxoSmithKline**
**Corporate Intellectual Property**
**Two New Horizons Court**
**Brentford, Middlesex TW8 9EP (GB)**

(56) References cited:
- **JOURNAL OF THE CHEMICAL SOCIETY, CHEMICAL COMMUNICATIONS. no. 15, 1970,LETCHWORTH GB pages 915 - 916; R.P.GLINSKI ET AL.: 'The Synthesis ofPhosphorylated 3'-Amino-3'-deoxythymidine and 5'-amino-5'-deoxythymidine'**
- **JOURNAL OF MEDICINAL CHEMISTRY. vol. 21, no. 1, 1978, WASHINGTON US pages 109 -112; T-S.LIN ET AL.: 'Synthesis and Biological Activity of Several AminoAnalogues of Thymidine'**
- **JOURNAL OF MEDICINAL CHEMISTRY. vol. 26, no. 12, 1983, WASHINGTON US pages 1691 - 1696; T.-S. LIN ET AL.: "Synthesis and Biological Activity of Various 3'-Azido and 3'-Amino Analogues of 5-Substituted Pyrimidine Deoxyribonucleodides"**
- **JOURNAL OF ORGANIC CHEMISTRY. vol. 38, no. 25, 1973, EASTON US pages 4299 -4305; R.P.GLINSKI ET AL.: 'Nucleotide Synthesis. IV. Phosphorylated 3'-Amino-3'-Deoxythymidine and 5'-Amino-5'-deoxythymidine and Derivatives'**
- **CHEMICAL ABSTRACTS, vol. 89, 1978, Columbus, Ohio, US; abstract no. 2214J, G.ROESLER ET AL.: '3'-Modified Nucleotides: Substrate Recognition byPhosphohydrolases' page 209 ;column 2 ;**
- **CHEMICAL ABSTRACTS, vol. 101, 1984, Columbus, Ohio, US; abstract no. 38769Z, V.E.ZAITSEVA ET AL.: 'Aminonucleosides and their Derivatives. Part X. 2'-Deoxydinucleoside Phosphates and 2'-Deoxydinucleotides with Phosphoamide Bonds'page 542 ;column 1 ;**
- **NUCLEIC ACID SYMPOSIUM SERIES vol. 9, 1981, LONDON pages 203 - 205; A.A.KRAEVSKII ET AL.: 'Synthesis of Oligonucleotides with 5'-3' phosphoamidoesterBond'**

- EXPERIMENTAL CELL RESEARCH vol. 116, no. 1, 1978, SWEDEN pages 21 - 29; C.J.KRIEG ET AL.: 'Increase in Intracisternal A-Type Particles in Friend CellsDuring Inhibition of Friend Virus (SFFV) Release by Interferon orAzidothymidine'
- EXPERIMENTAL CELL RESEARCH vol. 116, no. 1, 1978, SWEDEN pages 31 - 37; W.OSTERTAG: 'Induction of Intracisternal A-Type Particles During Friend CellDifferentiation'

## Description

The present invention relates to pharmaceutically acceptable derivatives of 3'-azido-3'-deoxythymidine and their use in the treatment or prophylaxis of human retroviral infections.

Retroviruses form a sub-group of RNA viruses which, in order to replicate, must first 'reverse transcribe' the RNA of their genome into DNA ('transcription' conventionally describes the synthesis of RNA from DNA). Once in the form of DNA, the viral genome is incorporated into the host cell genome, allowing it to take full advantage of the host cell's transcription/translation machinery for the purposes of replication. Once incorporated, the viral DNA is virtually indistinguishable from the host's DNA and, in this state, the virus may persist for as long as the cell lives. As it is virtually invulnerable to attack in this form, any treatment must be directed at another state of the life cycle and will, of necessity, have to be continued until all virus-carrying cells have died.

A species of retrovirus has now been reproducibly isolated from patients with AIDS (Acquired Immune Deficiency Syndrome). While it has been extensively characterised, there is, as yet, no agreed name for the virus, and it is currently known either as human T-cell lymphotropic virus III (HTLV III), AIDS-associated retrovirus (ARV), or lymphadenopathy associated virus (LAV). It is anticipated that the name to be agreed on internationally is acquired immune deficiency virus (AIDV). This virus (referred to herein as AIDV) has been shown preferentially to infect and destroy T-cells bearing the $OKT^4$ surface marker and is now generally accepted as the aetiologic agent of AIDS. The patient progressively loses this set of T-cells, upsetting the overall balance of the immune system, reducing his ability to combat other infections, and predisposing him to opportunistic infections which frequently prove fatal. Thus, the usual cause of death in AIDS victims is by opportunistic infection, such as pneumonia or virally induced cancers, and not as a direct result of AIDV infection.

Recently, AIDV has also been recovered from other tissue types, including B- cells expressing the $T^4$ marker, macrophages and non-blood associated tissue in the central nervous system (CNS). This latter infection has been discovered in patients expressing classical AIDS symptoms and is associated with progressive demyelination, leading to wasting and such symptoms as encephalopathy, progressive dysarthria, ataxia and disorientation.

There are at least four clinical manifestations of AIDV infection. In the initial 'carrier' state, the only indication of infection is the presence of anti-AIDV antibodies in the blood-stream. It is believed that such 'carriers' are capable of passing on the infection, e.g. by blood transfusion, sexual intercourse or used syringe needles. The carrier state may often never progress to the second stage characterized by persistent generalized lymphadenopathy (PGL). It is currently estimated that about 20% of PGL patients progress to a more advanced condition known as 'AIDS related complex' (ARC). Physical symptoms associated with ARC may include general malaise, increased temperature and chronic infections. This condition usually progresses to the final, fatal AIDS condition, when the patient completely loses the ability to fight infection.

The existence of these human retroviruses and others has only recently been recognized and, as the diseases with which they are linked are of a life-threatening nature, there exists an urgent need to develop ways to combat these viruses.

Various drugs have now been proposed as 'cures' for AIDS. These include antimoniotungstate, suramin, ribavirin and isoprinosine, which are either somewhat toxic or have shown no marked anti-retroviral activity. As the AIDV genome is incorporated into the host cell DNA after infection and is virtually invulnerable to attack in this state, it will persist as long as the host cell survives, causing new infection in the meantime. Thus, any treatment of AIDS would have to be for an extended period, possibly life, requiring substances with an acceptable toxicity.

Reports have described the testing of compounds against various retroviruses, for example, Friend Leukaemia Virus (FLV), a murine retrovirus. For instance Krieg et al. (Exp. Cell Res., 116 (1978) 21-29) found that 3'-azido-3'-deoxythymidine affected the release of C-type particles and increased the formation of A-type particles of FLV complex in vitro experiments, and Ostertag et al. (Proc. Nat. Acad. Sci. (1974) 71, 4980-85) stated that on the basis of antiviral activity related to the above FLV complex and a lack of cellular toxicity, 3'-azido-3'-deoxythymidine "might favourably replace bromodeoxyuridine for medical treatment of diseases caused by DNA viruses". However, De Clerq et al. (Biochem. Pharm. (1980) 29, 1849- 1851) established, six years later, that 3'-azido-3'-deoxythymidine had no appreciable activity against any viruses used in their tests, including vaccinia, HSVI and varicella zoster virus (VZV). Glinski et al. (J. Org. Chem. (1973), 38, 4299-4305) discloses certain derivatives of 3'-azido-3'-deoxythymidine (infra) and their ability to block mammalian exoribonuclease activity.

In a first aspect of the present invention, there are provided pharmaceutically acceptable derivatives of the compound of formula (I):

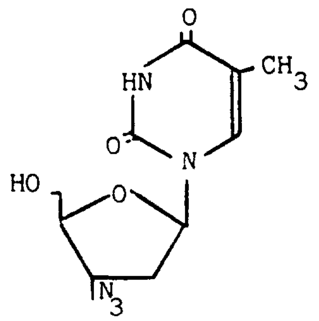

(I)

(i.e. 3'-azido-3'-deoxythymidine), for use in the treatment or prophylaxis of human retrovirus infections. The said derivatives of the compound of formula (I) are hereafter referred to as the compounds according to the invention.

Activity of 3'-azido-3'-deoxythymidine against human retroviruses has been established in various in vitro assay systems. For example, infection of the H9 human lymphoblastoid cell-line by AIDV is effectively prevented by concentrations of 3'-azido-3'-deoxythymidine as low as 0.013 µg/ml up to 20 hours after infection. AIDV infection of U937 human lymphoblastoid cells, PHA- stimulated white blood cells and cultured peripheral blood lymphocytes is also prevented at similarly low concentrations. In addition, 10-day challenge experiments using up to 5000 AIDV virions per cell and cloned T4, tetanus- specific, T-helper lymphocytes, showed no decrease in cells treated with 3'-azido-3'-deoxythymidine, while untreated cells had decreased 5-fold. Cytopathic effects were also completely blocked in the same cell-line transformed by HTLV-I and super-infected with AIDV.

Other studies using purified AIDV reverse transcriptase have shown that the activity of this enzyme is blocked by the triphosphate of 3'-azido-3'-deoxythymidine by a competitive inhibition mechanism. Phase I clinical trials have also shown that 3'-azido-3'-deoxythymidine is capable of crossing the blood/brain barrier in clinically effective quantities. This property is both unusual and valuable for the treatment and prophylaxis of CNS infections caused by human retroviruses.

The ability of 3'-azido-3'-deoxythymidine to modify the course of retrovirus- induced malignancy has been demonstrated in a mouse model, whereby administration of 3'-azido-3'-deoxythymidine prevented splenomegaly caused by intravenously administered Rauscher Murine Leukaemia Virus, the murine equivalent of HTLV-I. In further experiments, 3'-azido-3'-deoxythymidine has been shown to inhibit the in vitro replication of HTLV-I at concentrations as low as 0.8 µg/ml.

Thus, in a further, preferred aspect of the present invention, there is provided the use of a compound according to the invention in the manufacture of a medicament for the treatment or prophylaxis of human retrovirus infections.

Examples of human retrovirus infections which may be treated or prevented in accordance with the present invention include T-cell lymphotropic retroviruses (HTLV), especially HTLV-I, HTLV-II and AIDV (HTLV-III). Clinical conditions that may be treated or prevented in accordance with the invention include AIDS, AIDS-related complex and HTLV-I positive leukaemia and lymphoma. Suitable patients for treatment also include those having antibodies to AIDV, AIDV CNS infections, PGL and ARC.

By "a pharmaceutically acceptable derivative" is meant any pharmaceutically acceptable salt, ester, or salt of such ester, or any other compound which, upon administration to a human subject, is capable of providing (directly or indirectly) 3'-azido-3'-deoxythymidine or an anti-retrovirally active metabolite or residue thereof. An example of a non-ester compound is the derivative wherein the 5'-C- and 2-C-atoms are linked by an oxygen atom to form an anhydro group.

Preferred esters of the compound of formula (I) include carboxylic acid esters in which the non-carbonyl moiety of the ester grouping is selected from straight or branched chain alkyl, alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl); and mono-, di- or tri-phosphate esters. With regard to the above-described esters, unless otherwise specified, any alkyl moieties present in such esters advantageously contain 1 to 18 carbon atoms, particularly 1 to 4 carbon atoms. Any aryl moiety present in such esters advantageously comprises a phenyl group. Any reference to any of the above compounds also includes a reference to a pharmaceutically acceptable salt thereof.

Experiments have shown that 3'-azido-3'-deoxythymidine is converted in vivo, by the action of cellular enzymes into the 5'-monophosphate. The monophosphate is then further phosphorylated by other enzymes to form the triphosphate via the diphosphate, and other studies have demonstrated that it is the triphosphate form of 3'-azido-3'-deoxythymidine which is believed to be the effective chain terminator in the reverse transcription of AIDV, as evidenced by its effect on avian myeloblastosis virus and Moloney murine leukaemia virus. This form also inhibits AIDV reverse

transcriptase in vitro whilst having a negligible effect on human DNA polymerase activity.

Examples of pharmaceutically acceptable salts of the compound of formula (I) and its pharmaceutically acceptable derivatives include base salts, eg derived from an appropriate base, such as alkali metal (e.g. sodium), alkaline earth metal(e.g. magnesium) salts, ammonium and $NX_4^+$ (wherein X is $C_{1-4}$ alkyl).

The present invention thus further provides the novel pharmaceutically acceptable derivatives of the compound of formula (I), other than the following 5'-derivatives, namely monophosphate, disodium monophosphate, 2-cyanoethyl monophosphate, monosodium 2-cyanoethyl monophosphate, 4-nitrophenyl monophosphate, triphosphate, p-toluene sulphonate, acetate, triphenylmethyl and methanesulphonate derivatives or where the 5' C is linked to a further nucleotide or nucleoside derivative.

Specific examples of pharmaceutically acceptable derivatives of the compound of formula (I) that may be used in accordance with the present invention include the monosodium salt and the following 5' esters: monophosphate; disodium monophosphate; diphosphate; triphosphate; acetate; 3-methyl-butyrate; octanoate; palmitate; 3-chloro benzoate; benzoate; 4-methyl benzoate; hydrogen succinate; pivalate; and mesylate.

The compounds according to the invention may be administered to humans for prophylaxis or treatment of retroviral infections by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the compound according to the invention.

In general a suitable dose will be in the range of 3.0 to 120 mg per kilogram body weight of the patient per day, preferably in the range of 6 to 90 mg per kilogram body weight per day and most preferably in the range 15 to 60 mg per kilogram body weight per day. The desired dose is preferably presented as two, three, four, five, six or or more sub-doses administered at appropriate intervals throughout the day. These sub-doses may be administered in unit dosage forms, for example, containing 5 to 1500 mg, preferably 10 to 1000 mg, and most preferably 20 to 700 mg of active ingredient per unit dosage form.

Experiments with 3'-azido-3'-deoxythymidine suggest that a dose of the compound according to the invention should be administered to achieve peak plasma concentrations of the said compound of from about 1 to about 75 μM, preferably about 2 to 50 μM, most preferably about 3 to about 30 μM. This may be achieved, for example, by the intravenous injection of a 0.1 to 5% solution of the compound, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the compound.

While it is possible for the compound according to the invention to be administered alone it is preferable to present it as a pharmaceutical formulation. The formulations of the present invention comprise at least one compound according to the invention, as above defined, together with one or more acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the compound with the carrier which constitutes one or more accessory ingredients. In general, the formulations are prepared by uniformly and intimately bringing into association the compound according to the invention with liquid carriers or finely divided solid carriers or both, and then if necessary shaping the product.

Formulations of the present invention suitable for oral administration may be presented as discrete units such as capsules, cachets or tablets each containing a predetermined amount of the compound according to the invention; as a powder or granules; as a solution or a suspension in an aqueous or non-aqueous liquid; or as an oil-in-water liquid emulsion or a water-in-oil liquid emulsion. The compound according to the invention may also be presented as a bolus, electuary or paste. Oral formulations may further include other agents conventional in the art, such as sweeteners, flavouring agents and thickeners. The above formulations also include those providing sustained release of the active ingredient after oral administration.

A tablet may be made by compression or moulding, optionally with one or more accessory ingredients. Compressed tablets may be prepared by compressing in a suitable machine the compound according to the invention in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide the desired release profile.

Formulations suitable for topical administration in the mouth include lozenges comprising the compound according to the invention in a flavoured basis, usually sucrose and acacia or tragacanth; pastilles comprising the active ingredient in an inert basis such as gelatin and glycerin, or sucrose and acacia; and mouthwashes comprising the compound in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the compound according to the invention such carriers as are known in the art to be appropriate.

Formulations suitable for parenteral administration include aqueous and non- aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of the compound according to the invention.

The compounds according to the invention may also be used in therapy in conjunction with other medicaments such as 9-[[2-hydroxy-1-(hydroxymethyl)ethoxy]methyl]guanine, 9-(2-hydroxyethoxymethyl)-guanine (acyclovir), 2-amino-9-(2-hydroxyethoxymethyl)purine, interferon, e.g., μ-interferon, interleukin II, and phosphonoformate (Foscarnet) or in conjunction with other immune modulating therapy including bone marrow or lymphocyte transplants or medications such as levamisol or thymosin which serve to increase lymphocyte numbers and/or function as is appropriate.

It should be understood that in addition to the ingredients particularly mentioned above the formulations of this invention may include other agents conventional in the art of formulation.

The compounds according to the invention may be prepared in conventional manner, for example as described in the following references, or by methods analogous thereto:J.R. Horwitz et al., J. Org. Chem. 29, (July 1964) 2076-78; M. Imazawa et al., J. Org. Chem 43 (15) (1978) 3044-3048; K.A. Watanabe et al., J. Org. Chem., 45, 3274 (1980); and R.P. Glinski et al., J. Chem. Soc. Chem. Commun., 915 (1970), as well as the processes described in the Examples.

The present invention further includes a process for the preparation of a compound according to the invention which comprises

(A) reacting a compound of formula:

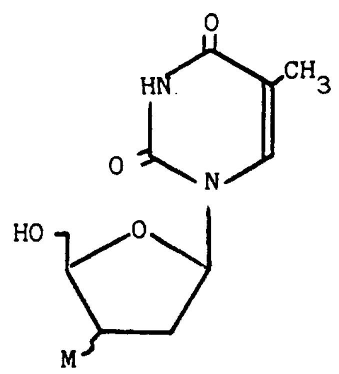

(II)

(wherein M represents a precursor group for the 3' -azido group) or a derivative (e.g. an ester or salt) thereof, with an agent or under conditions serving to convert the said precursor group into the desired azido group; or

(B) reacting a compound of formula:

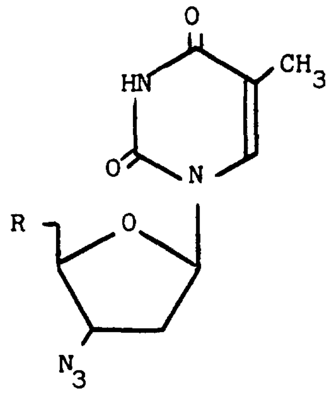

(III)

(wherein R represents a precursor group for the hydroxy group, or for a pharmaceutically acceptable derivative group thereof) with an agent or under conditions serving to convert the said precursor group into the corresponding desired group; or

(C) reacting a compound of formula

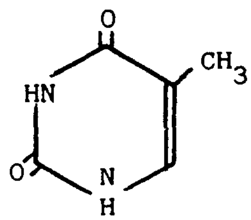

(IV)

or a functional equivalent thereof, with a compound serving to introduce the desired ribofuranosyl ring at the 1-position of the compound of formula (IV); or

(D) reacting a compound of formula

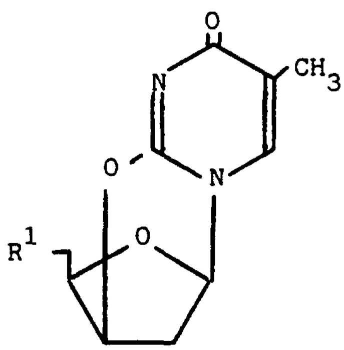

(V)

(wherein $R^1$ is hydroxy or R as defined above), with an agent or under conditions serving to convert the said compound into a compound according to the invention;

and thereafter, or simultaneously therewith, effecting one or more of the following conversions:-

(i) when 3'-azido-3'-deoxythymidine is formed, converting it into a pharmaceutically acceptable derivative thereof,
(ii) when a pharmaceutically acceptable derivative of 3'-azido-3'-deoxythymidine is formed, optionally converting the said derivative into a different such derivative.

In the above-described process according to the invention, it will be appreciated that the precursor compounds

of formulae (II) and (III), as well as the above- mentioned agents and conditions, will be selected from those that are known in the art of nucleoside synthetic chemistry. Examples of such conversion procedures are described hereinafter for guidance and it will be understood that they can be modified in conventional manner depending on the desired compound of formula (I). In particular, where a conversion is described which would otherwise result in the undesired reaction of labile groups then such groups may be protected in conventional manner, with subsequent removal of the protecting groups after completion of the conversion. Thus, in process (A), the group M in the compound of formula (II) may represent, for example, a halogen (e.g. chlorine), hydroxy or organosulphonyloxy e.g. trifluoromethylsulphonyloxy, methanesulphonyloxy or p-toluene sulphonyloxy radical.

For the preparation of the compound of formula (I), a compound of formula (II) in which the group M is a halogen (eg chloro) group in the threo configuration (in which the 5'-hydroxy is advantageously protected, eg with a trityl group) may be treated for example with lithium or sodium azide. The 3'- threo-halogen (eg chlorine) starting material may be obtained for example by reaction of the corresponding 3'-erythro-hydroxy compound with for example triphenylphosphine and carbon tetrachloride, or alternatively by treatment with organosulphonyl halide (eg trifluoromethanesulphonyl chloride) to form a corresponding 3'- erythro-organosulphonyloxy compound which is then halogenated. Alternatively a 3'-threo-hydroxy compound of formula (II) may be treated, for example with triphenylphosphine, carbon tetrabromide and lithium azide to form the corresponding 3'-erythro azido compound. Removal of any protecting group may then subsequently be effected, e.g. as above.

With regard to process (B), R may represent a protected hydroxy group e.g. an ester grouping of the type referred to above in relation to formula (I) particularly acetoxy, or an ether group such as a trialkylsilyloxy group, e.g. t- butyldimethylsilyloxy or an aralkoxy group e.g. triphenylmethoxy. Such groups may be converted for example by hydrolysis to the desired hydroxy group or, by transesterification, be converted to an alternative ester group of 3'-azido-3'- deoxythymidine.

With regard to process (C), this may be effected for example by treating the appropriate pyrimidine of formula (IV) or a salt or protected derivative thereof, with a compound of formula (wherein A represents a leaving group, e.g. an acetoxy or benzoyloxy or halo, eg chloro group and B represents an optionally protected hydroxy group eg a p-toluenesulphonyloxy group), and subsequently removing any protecting groups.

Regarding process (D), $R^1$ may represent a precursor group as described above for R in formula (III). 3'-Azido-3'-deoxythymidine may then, for example, be obtained by reaction with an alkali metal azide, e.g. lithium azide, advantageously in an appropriate solvent such as moist DMF followed by acid or base hydrolysis advantageously under mild conditions.

3'-Azido-3'-deoxythymidine may be converted into a pharmaceutically acceptable phosphate or other ester by reaction with respectively a phosphorylating agent, e.g. $POCl_3$ or an appropriate esterifying agent, e.g. an acid halide or anhydride. The compound of formula (I), including esters thereof, may be converted into pharmaceutically acceptable salts thereof in conventional manner, e.g. by treatment with an appropriate base. An ester or salt of 3'-azido- 3'-deoxythymidine may be converted into the parent compound, e.g. by hydrolysis.

The following Examples are intended for illustration only and are not intended to limit the scope of the invention in any way. The term 'active ingredient' as used in the Examples means a pharmaceutically acceptable derivative of the compound of formula (I).

Example 1: Tablet Formulations

The following formulations A to C were prepared by wet granulation of the ingredients with a solution of povidone, followed by addition of magnesium stearate and compression.

Formulation A

| | | mg/tablet | mg/tablet |
|---|---|---|---|
| (a) | Active ingredient | 250 | 250 |
| (b) | Lactose B.P. | 210 | 26 |
| (c) | Povidone B.P. | 15 | 9 |
| (d) | Sodium Starch Glycollate | 20 | 12 |
| (e) | Magnesium Stearate | 5 | 3 |
| | | 500 | 300 |

Formulation B

| | | mg/tablet | mg/tablet |
|---|---|---|---|
| (a) | Active ingredient | 250 | 250 |
| (b) | Lactose | 150 | - |
| (c) | Avicel PH 101 | 60 | 26 |
| (d) | Povidone B.P. | 15 | 9 |
| (e) | Sodium Starch Glycollate | 20 | 12 |
| (f) | Magnesium Stearate | 5 | 3 |
| | | 500 | 300 |

Formulation C.

| | mg/tablet |
|---|---|
| Active ingredient | 100 |
| Lactose | 200 |
| Starch | 50 |
| Povidone | 5 |
| Magnesium stearate | 4 |
| | 359 |

The following formulations, D and E, were prepared by direct compression of the admixed ingredients. The lactose used in formulation E was of the direct compression type (Dairy Crest - "Zeparox").

Formulation D

| | mg/tablet |
|---|---|
| Active Ingredient | 250 |
| Pregelatinised Starch NF15 | 150 |
| | 400 |

Formulation E

| | mg/tablet |
|---|---|
| Active Ingredient | 250 |
| Lactose | 150 |
| Avicel | 100 |
| | 500 |

Formulation F (Controlled Release Formulation)

The formulation was prepared by wet granulation of the ingredients (below) with a solution of povidone followed by the addition of magnesium stearate and compression.

| | | mg/tablet |
|---|---|---|
| (a) | Active Ingredient | 500 |
| (b) | Hydroxypropylmethylcellulose (Methocel K4M Premium) | 112 |
| (c) | Lactose B.P. | 53 |
| (d) | Povidone B.P.C. | 28 |
| (e) | Magnesium Stearate | 7 |
| | | 700 |

Drug release took place over a period of about 6-8 hours and was complete after 12 hours.

Example 2: Capsule Formulations

Formulation A

A capsule formulation was prepared by admixing the ingredients of Formulation D in Example 1 above and filling into a two-part hard gelatin capsule. Formulation B (infra) was prepared in a similar manner.

Formulation B

| | | mg/capsule |
|---|---|---|
| (a) | Active ingredient | 250 |
| (b) | Lactose B.P. | 143 |
| (c) | Sodium Starch Glycollate | 25 |
| (d) | Magnesium Stearate | 2 |
| | | 420 |

Formulation C

| | | mg/capsule |
|---|---|---|
| (a) | Active ingredient | 250 |
| (b) | Macrogol 4000 BP | 350 |
| | | 600 |

Capsules were prepared by melting the Macrogol 4000 BP, dispersing the active ingredient in the melt and filling the melt into a two-part hard gelatin capsule.

Formulation D

| | mg/capsule |
|---|---|
| Active ingredient | 250 |
| Lecithin | 100 |
| Arachis Oil | 100 |
| | 450 |

Capsules were prepared by dispersing the active ingredient in the lecithin and arachis oil and filling the dispersion into soft, elastic gelatin capsules.

Formulation E (Controlled Release Capsule)

The following controlled release capsule formulation was prepared by extruding ingredients a, b and c using an extruder, followed by spheronisation of the extrudate and drying. The dried pellets were then coated with release-controlling membrane (d) and filled into a two-piece, hard gelatin capsule.

| | | mg/capsule |
|---|---|---|
| (a) | Active Ingredient | 250 |
| (b) | Microcrystalline Cellulose | 125 |
| (c) | Lactose BP | 125 |
| (d) | Ethyl Cellulose | 13 |
| | | 513 |

Example 3: Injectable Formulation

Formulation A.

| | |
|---|---|
| Active ingredient | 0.200g |
| Hydrochloric acid solution, 0.1 M q.s. to pH | 4.0 to 7.0 |
| Sodium hydroxide solution, 0.1 M q.s. to pH | 4.0 to 7.0 |
| Sterile water q.s. to | 10ml |

The active ingredient was dissolved in most of the water (35&-40&C) and the pH adjusted to between 4.0 and 7.0 with the hydrochloric acid or the sodium hydroxide as appropriate. The batch was then made up to volume with the water and filtered through a sterile micropore filter into a sterile 10ml amber glass vial (type 1) and sealed with sterile closures and overseals.

Formulation B.

| | |
|---|---|
| Active ingredient | 0.125 g |
| Sterile, pyrogen-free, pH 7 phosphate buffer, q.s. to | 25 ml |

Example 4: Intramuscular injection

| | |
|---|---|
| Active Ingredient | 0.20 g |
| Benzyl Alcohol | 0.10 g |
| Glycofurol 75 | 1.45 g |
| Water for Injection q.s. to | 3.00 ml |

The active ingredient was dissolved in the glycofurol. The benzyl alcohol was then added and dissolved, and water added to 3 ml. The mixture was then filtered through a sterile micropore filter and sealed in sterile 3 ml amber glass vials (type 1).

Example 5: Ingredients

| | |
|---|---|
| Active ingredient | 0.2500 g |

(continued)

| | |
|---|---|
| Sorbitol Solution | 1.5000 g |
| Glycerol | 2.0000 g |
| Sodium Benzoate | 0.0050 g |
| Flavour, Peach 17.42.3169 | 0.0125 ml |
| Purified Water q.s. to | 5.0000 ml |

The active ingredient was dissolved in a mixture of the glycerol and most of the purified water. An aqueous solution of the sodium benzoate was then added to the solution, followed by addition of the sorbitol solution and finally the flavour. The volume was made up with purified water and mixed well.

Example 6: Suppository

| | mg/suppository |
|---|---|
| Active Ingredient (63µm)* | 250 |
| Hard Fat, BP (Witepsol H15 - Dynamit NoBel) | 1770 |
| | 2020 |

*The active ingredient was used as a powder wherein at least 90% of the particles were of 63µm diameter or less.

One-fifth of the Witepsol H15 was melted in a steam-jacketed pan at 45&C maximum. The active ingredient was sifted through a 200#m sieve and added to the molten base with mixing, using a silverson fitted with a cutting head, until a smooth dispersion was achieved. Maintaining the mixture at 45°C, the remaining Witepsol H15 was added to the suspension and stired to ensure a homogenous mix. The entire suspension was passed through a 250#m stainless steel screen and, with continuous stirring, was allowed to cool to 40°C. At a temperature of 38°C to 40°C 2.02g of the mixture was filled into suitable plastic moulds. The suppositories were allowed to cool to room temperature.

Example 7: Pessaries

| | mg/pessary |
|---|---|
| Active ingredient 63µm | 250 |
| Anhydrate Dextrose | 380 |
| Potato Starch | 363 |
| Magnesium Stearate | 7 |
| | 1000 |

The above ingredients were mixed directly and pessaries prepared by direct compression of the resulting mixture.

Example 8: 3'-Azido-3'-deoxy-5'-0-octanoylthymidine

To a solution of 3'-azido-3'-deoxy thymidine in pyridine (0°C), octanoyl chloride (1.2 equivalents) was added. The reaction was allowed to warm to room temperature. When tlc ($CHCl_3$:MeOH;20:1, on silica gel) indicated complete reaction, the solution was poured onto ice water. The aqueous phase was decanted. The resulting oil was chromatographed on silica gel eluted with $CHCl_3$:MeOH. The title compound was obtained as an oil by evaporation of the solvent from the appropriate fractions.

| | | | | |
|---|---|---|---|---|
| CHN: | cal. | C-54.95 | H-6.92 | N-17.80 |
| | fnd. | C-54.82 | H-6.96 | N-17.66 |

δ7.46(d,1H,$J_{5,6}$=1Hz,6H), δ6.13(t,1H,1'H), δ4.5-4.2(m,3H,3'H and 5'$CH_2$) δ4.0-3.8(m,1H,4'H), δ2.3-2.1-(m,4H,2'H and ($CH_2$)l of octanoyl)), δ1.81(d,3H,$J_{5,6}$=1.0Hz, 5$CH_3$), δ1.5-0.6(m,13H,5' octanoyl $(CH_2)_5CH_3$)

Example 9: 5'-Acetyl-3'-azido-3'-deoxythymidine

To a solution of 3'-azido-3'-deoxythymidine (20g) in pyridine (50ml) at ambient temperature, acetyl chloride (2.1 equivalents) was added. The reaction was stirred for two hours and kept at 0 to 5°C for 20 hours. It was poured onto ice water with stirring. The aqueous phase was decanted. The oily product was dissolved in water and extracted with water (5 times), 0.5 N hydrochloric acid, water (2x), and dried over magnesium sulphate. The solution was filtered and evaporated in vacuo. The residual oil was dissolved in chloroform, applied to a silica gel column, and flash chromatographed using 2% methanol in chloroform. Fractions with product were evaporated and the oil was chromatographed again using ethyl acetate: hexane (6:4 v/v). Fractions with product were evaporated in vacuo to a white solid. m.p. 96-98°C

| | | | |
|---|---|---|---|
| cal. | C-46.60 | H-4.89 | N-22.65 |
| fnd. | C-46.67 | H-4.94 | N-22.59 |

Example 10

The following compounds were prepared according to the procedure of Example 8 or 9 as appropriate from the appropriate acid halide or anhydride.

| | | | | |
|---|---|---|---|---|
| 3'-Azido-5'-0-benzoyl-3'-deoxythymidine | cal. | C-53.68 | H-4.77 | N-18.41 |
| | fnd. | C-53.81 | H-4.72 | N-18.46 |
| | m.p. 54-59°C | | | |

| | | | | |
|---|---|---|---|---|
| 3'-Azido-3'-deoxy-5'-0-pivaloylthymidine | cal. | C-51.27 | H-6.03 | N-19.93 |
| | fnd. | C-51.07 | H-6.05 | N-19.83 |
| | m.p. 99-100°C | | | |

| | | | | |
|---|---|---|---|---|
| 3'-Azido-3'-deoxy-5'-0-(3-methylbutyryl)thymidine | cal. | C-50.24 | H-6.13 | N-19.53 |
| | fnd. | C-50.27 | H-6.11 | N-19.49 |

δ7.46(d,1H,$J_{5,6}$=1.2Hz,6H), δ6.13(t,1H,1'H), δ4.55-4.15(m,3H,3'H and 5'CH2), δ3.8-4.15(m,1H,4'H),δ2.4-1.78-(m,3H, 2'H and 5'methine), δ1.80(d,3H,$J_{5,6}$=1.2Hz,5$CH_3$), δ0.9(d,6H,J=6.4Hz, methyls on 5'butyryl)

| | | | | |
|---|---|---|---|---|
| 3'-Azido-3'-deoxy-5'-0-palmitoylthymidine | cal. | C-61.67 | H-8.57 | N-13.85 |
| | fnd. | C-61.85 | H-8.59 | N-13.75 |

δ7.45(d,1H,$J_{5,6}$=1.0Hz,6H), δ6.12(t,1H,1'H), δ4.5-4.05(m,3H,3'H and 5'$CH_2$), δ4.0-3.8(m,1H,4'H) δ2.35-2.11-(m,4H, 2'H and ($CH_2$)l of palmitoyl), δ1.8 d, 3H, $J_{5,6}$=1.0Hz,5$CH_3$) δ1.35-0.6(m,29H,5'palmitoyl$(CH_2)_{13}CH_3$)

| | | | | |
|---|---|---|---|---|
| 3'-Azido-3'-deoxy-5'-0-toluylthymidine | Cal. | C-56.10; | H-4.97; | N-18.17 |
| | Fnd. | C-55.88; | H-5.00; | N-18.09 |
| | m.p. 73°C | | | |

NMR taken in DMSO-$d_6$
NMR: δ7.95-7.29 (m,5H; bH,cH,6H), δ6.16 (t,1H,1'H), δ4.6-4.4 (m,3H,3'H,5'H), 84.2-4.0 (m,1H,4'H), δ2.39 (s,3H,d$CH_3$), δ1.63 (s,3H,5$CH_3$)

| | | | | |
|---|---|---|---|---|
| 3'-Azido-3'-deoxythymidine 5'-O-(hydrogen succinate) | Cal. | C-44.98; | H-4.83; | N-17.96 |
| | Fnd. | C-44.90; | H-4.77; | N-17.85 |

NMR taken in DMSO-$d_6$
NMR: δ7.46 (s,1H,6H), δ6.13 (m,1H,1'H), δ4.48-4.40 (m,1H,3'H), δ4.34-4.20 (m,2H,5'H), δ3.99-3.94 (m,1H,4'H), δ1.78 (s,3H,$5CH_3$)

| | | | | | |
|---|---|---|---|---|---|
| 3'-Azido-3'-deoxy-5'-mesylthymidine | Cal. | C-38.25; | H-4.37; | N-20.28; | S-9.28 |
| | Fnd. | C-38.15; | H-4.38; | N-20.19: | S-9.30 |
| | m.p. 253°C (dec.) | | | | |

NMR taken in DMSO-$d_6$
NMR: δ7.49 (d,1H,$J_{6,5}$ = 1.0 Hz, 6H), c6.15 (t,1H,$J_{1',2'}$ = 6.6 Hz, 1'H), δ4.54-4.41 (m,3H;3'H,5'H), δ4.14-4.02 (m,1H, 4'H), δ3.24 (s,3H,5'-mesyl $CH_3$),
δ1.79 (d, 3H,$J_{5,6}$ = 1.0 Hz, $5CH_3$)

| | | | | | |
|---|---|---|---|---|---|
| 3'-Azido-5'-O-(3-chlorobenzoyl)-3'-deoxythymidine | Cal. | C-50.31; | H-3.97; | N-17.26; | C1-8.74 |
| | Fnd. | C-50.16; | H-4.03; | N-17.13; | C1-8.66 |

NMR taken in DMSO-$d_6$
NMR: δ11.37 (s,1H,3-NH), δ7.98-7.43 (m,5H;5'-phenyl,6H), δ6.17 (dd,1H; $J_{1',2a'}$ = 6.1 Hz, $J_{1',2b'}$ = 7.2 Hz, 1'H), δ4.68-4.48 (m,3H;3'H,5'H), δ4.14-4.11 (m,1H,4'H), δ2.48-2.41 (m,2H,2'H), δ1.64 (d,3H,$J_{5,6}$ = 1.2 Hz, $5CH_3$)

Example 11: 2.5'-O-Anhydro-3'-azido-3'-deoxythymidine

3'-Azido-3'-deoxythymidine (3.0g, 11.2 mMol) was mesylated by the addition of methanesulphonyl chloride (2.7 mL) to a solution of the starting material in dry pyridine (2 mL). The reaction was allowed to proceed at 5°C for one hour, then poured onto ice water. The precipitate was collected by filtration. The product (3'-azido-5'- mesylthymidine) was reacted with potassium carbonate (0.78 g, 5.6 mMol) in DMF (75 mL). The reactants were heated in an 80°C oil bath for six hours, then poured into ice water. The product was extracted from the water with ethyl acetate. The solvent was removed in vacuo and the resultant oil was flash chromatographed on silica gel by elution with $CHCl_3$ : MeOH (9:1 v/v). The title compound was obtained as a solid after evaporation of the solvent from the appropriate fractions.
m.p. = 184 - 186°C

Reference Example: 3'-Azido-3'-deoxythymidine

a) 2.3'-Anhydrothymidine
Thymidine (85.4 g: 0.353 mol) was dissolved in 500 ml dry DMF and added to N- (2-chloro-1,1,2-trifluoroethyl) diethylamine (100.3 g; 0.529 mol) (prepared according to the method of D.E. Ayer, J. Med. Chem. 6, 608 (1963)). This solution was heated at 70°C for 30 minutes then poured into 950 ml ethanol (EtOH) with vigorous stirring. The product precipitated from this solution and was filtered. The EtOH supernatant was refrigerated then filtered to yield the title compound. mp. = 228 -230°C.

b) 3'-Azido-3'-deoxythymidine
2,3'-0-Anhydrothymidine (25 g: 0.1115 mol) and $NaN_3$ (29 g, 0.446 mol) was suspended in a mixture of 250 ml DMF and 38 ml water. The reaction mixture was refluxed for 5 hours at which time it was poured into 1 liter of water. The aqueous solution was extracted with EtOAc (3 x 700 ml). The EtOAc extracts were dried over $Na_2SO_4$, filtered and the EtOAc was removed in vacuo to yield a viscous oil. This oil was stirred with 200 ml water providing the title compound as a solid which was collected by filtration. mp = 116-118°C

Example 12: Monosodium Salt of 3'-Azido-3'-deoxythymidine

Approximately 1g of 3'-azido-3'-deoxythymidine was dissolved in 50mL of distilled water. The pH was adjusted to 12 with 1N NaOH. Approximately half of the solution was freeze-dried. The title compound was obtained as a lyophilised powder.

| Analysis calculated for $C_{10}H_{12}N_5NaO_4$ 6/10 $N_2O$ | | | | |
|---|---|---|---|---|
| cal: | C-40.03; | H-4.43; | N-23.34; | Na-7.66 |
| fnd: | C-39.88; | H-4.34; | N-23.23; | Na-7.90 |

Example 13: Preparation of 5'-Monophosphate of 3'-Azido-3'-deoxythymidine

3'-Azido-3'-deoxythymidine (0.5 g, 1.87 mmol) was dissolved in 5 mL of triethyl phosphate and the mixture was cooled to -5°C. Phosphorus oxychloride (0.685 mL, 7 mmol) was added in one portion to the rapidly stirred solution which was then maintained at -10°C for 22 hours. An aliquot was removed and added to concentrated ammonium hydroxide. Analysis of this sample on TLC (cellulose, n-PrOH:$H_2O$, 7:3 v/v) showed no remaining starting material and a single fluorescent spot with lower mobility than the nucleoside. The reaction mixture was poured onto 20mL of ice and water. This was placed in an ice bath and the pH of the solution was adjusted to a value of 7.5 by the addition of 2N NaOH. The basic mixture was extracted once with chloroform and once with ether. The aqueous layer was again adjusted to give a pH of 7.5 and concentrated in vacuo to remove residual organic solvent. The material was stored at -10°C until purified as follows:

Deactivated charcoal was prepared by washing coconut charcoal (50-200 mesh, 100 g) with 500 mL of 1 N HCl, 3 L of water, 35 mL of 3% toluene in 95% ethanol, 600 mL of 95% ethanol and finally extensively with water. Deactivated charcoal (12 mL of settled wet charcoal) was added with stirring to the monophosphate solution (0.72 g, 1.8 mmol, 30 mL). The supernatant was decanted and the charcoal was washed with 150 mL of water. The nucleotide was eluted from the charcoal by washing with 120 mL of 1.5 M ammonium hydroxide in 50% ethanol. This solution was filtered through a 0.22 micron filter, concentrated in vacuo to 10 mL, filtered through a Amicon Centriflo CF- 25 membrane, and lyophilized to yield the diammonium 3'- azido-3'- deoxythymidine-5'-monophosphate as a solid. This compound was characterized as a nucleoside 5'-monophosphate by the ability of 5'-nucleotidase to degrade it to the nucleoside.

Example 14: 3'-Azido-5'triphosphate-3'-deoxythymidine and-3'-Azido-5'-diphosphate-3'-deoxythymidine

(a) Bis (tri-n-butylammonium)pyrophosphate

A column of DOW 50 ('Dowex' ion exchange resin - DOW Chemical Laboratories) pyridinium resin was prepared by pouring 40 mL of resin into a 25 cm diameter column and washed with water until no more colour eluted. Pyrophosphate decahydrate (1.12 g, 2.51 mM) was dissolved in 30 mL of water and applied to the column. The column was eluted with water. A 125 mL fraction of the eluant which contained UV absorbing material was collected. The volume was reduced to 10 mL in vacuo and tri-n-butylamine (1.2 mL) was added. The volume was reduced in vacuo and the residue was dried by coevaporation with pyridine four times. The product was stored in a freezer (-5°C).

(b) Hydrogen Form of 3'-Azido-5'-monophosphate-3'-deoxythymidine

The hydrogen form of the monophosphate was prepared by passing the ammonium salt obtained in Example 14 (0.1 g, 0.283 mMol) dissolved in 6 mL of water, through a 1.5 mL (10 eq.) column of DOW 50 $H^+$.

(c) Phosphoromorpholidate Derivative of 3'-Azido-3'-deoxythymidine

In 9 mL of water was dissolved 0.283 mMol of the hydrogen form of the monophosphate obtained in stage b). Morpholine (99 #L, 1.13 mMol, 4 eq.) was added and the solution heated to reflux. Dicyclohexyl carbodiimide (0.234 g, 1.13 mMol, 4 eq.) dissolved in t-butanol (5 mL) was added over a three hour period. The reaction was refluxed overnight. The reaction was cooled to room temperature, filtered, and the solvents removed in vacuo. Ethanol was added and evaporated in vacuo four times. The residue was dissolved in methanol and the phosphoromorpholidate precipitated by the addition of ether. The precipitate was triturated with ether four times and dried on a rotary evaporator. The title compound was obtained.

(d) 3'-Azido-3'-deoxythymidine-5'-triphosphate

The phosphoromorpholidate derivative obtained in stage c), was dried by a removal of pyridine in vacuo four times. The bis (n-Bu)$_3$N pyrophosphate obtained in stage a) was also dried by removal of pyridine in vacuo. The

phosphoromorpholidate was dissolved in pyridine, 5 mL, and added to the vessel containing the pyrophosphate reagent. The reaction was allowed to continue overnight at room temperature. The pyridine was removed in vacuo. Water was added to the residue and removed in vacuo three times. The residue was frozen.

The residue was thawed and dissolved in 50 mL of water. The solution was applied to a column (1 x 10 cm) of DEAE Saphadex A-25 which had been equilibrated with 50 mM ammonium bicarbonate. The phosphates were eluted with a 300 mL linear gradient of 50-800 mM ammonium bicarbonate. The fractions containing the diphosphate nucleotide were pooled as were those containing the triphosphate nucleotide. The pooled diphosphate and triphosphate fractions were each dried in vacuo. redissolved in water, dried again, redissolved in water and lyophilized.

Example 15: Enzymatic Synthesis of 3'-Azido-5'-triphosphate-3'-deoxythymidine

The 5'-triphosphate was synthesized from the 5'-diphosphate using pyruvate kinase and nucleoside diphosphate kinase. The reaction mixture contained: 6mM 3'-azido TDP, 12 mM adenosine triphosphate, 40 mM $MgCl_2$, 40 mM potassium piperazine-N,N'-bis(2-ethanesulphonic acid) PIPES buffer (pH 6.8), 30 mM phosphoenolpyruvate, 40 IU/ml nucleoside diphosphate kinase and 100 IU/ml pyruvate kinase in a final volume of 5 mL. The reaction mixture was incubated at 37°C for 5 days. The reaction mixture was applied to a column (2.5 x 10 cm) of DEAE Sephadex A-25 which had been equilibrated with ammonium bicarbonate. The nucleotides were eluted with a gradient of 100 -1000 mM ammonium bicarbonate. Fractions containing the triphosphate were pooled, and evaporated to dryness in vacuo. The compound was further purified using a preparative HPLC column (Whatman, Inc., Magnum 9 SAX) eluted with a gradient of 10 - 100 mM potassium phosphate, pH 3.5. The resulting compound was further purified using a DEAE Sephadex A-25 column as above. The fractions containing the tetraammonium 3'-azido-3'-deoxythymidine-5'- triphosphate were pooled, dried in vacuo, redissolved in water and lyophilized to yield the title compound.

## Claims

### Claims for the following Contracting States : BE, CH, DE, FR, GB, IT, LI, LU, NL, SE

**1.** A pharmaceutical formulation comprising a pharmaceutically acceptable derivative of 3'-azido-3'-deoxythymidine, and a pharmaceutically acceptable carrier therefore, said formulation comprising a unit dose of between 5 and 1500mg of the active ingredient.

**2.** A formulation according to claim 1 wherein the carrier is other than water.

**3.** A formulation according to claim 1 or 2 which is sterile.

**4.** A formulation according to claim 3 adapted for administration by injection.

**5.** A formulation according to claim 4 contained in a sealed vial.

**6.** A formulation according to either of claims 3 and 4 wherein the carrier is sterile water.

**7.** A formulation according to claim 1 or 2 adapted for oral administration.

**8.** A formulation according to any of claims 1, 2 or 7 in the form of a tablet or capsule.

**9.** A formulation according to any of claims 1, 2 or 3 providing sustained release of the active ingredient after oral administration.

**10.** A formulation according to claim 7 further comprising a flavouring agent.

**11.** A formulation according to any of claims 1 to 10 wherein the unit dose is between 10 and 1000 mg of the active ingredient.

**12.** A formulation according to any of claims 1 to 10 wherein the unit dose is between 20 and 700 mg of the active ingredient.

13. A process for the preparation of a formulation according to any one of claims 1 to 12 comprising bringing a pharmaceutically acceptable derivative of 3'-azido-3'-deoxythymidine into association with the said pharmaceutically acceptable carrier.

**Claims for the following Contracting State : AT**

1. A process for the preparation of a pharmaceutical formulation comprising bringing a pharmaceutically acceptable derivative of 3'-azido-3'-deoxythymidine as active ingredient, into association with a carrier therefore, other than to form a simple, non-sterile aqueous solution of the active ingredient, wherein the active ingredient is presented in a unit dose of between 5 and 1500mg.

2. A process for the preparation of a formulation according to claim 1 wherein the carrier is other than water.

3. A process for the preparation of a formulation according to claim 1 or 2 wherein the ingredients are sterile.

4. A process for the preparation of a formulation to claim 3 wherein said formulation is adapted for administration by injection.

5. A process for the preparation of a formulation according to claim 4 further comprising storing in a sealed vial.

6. A process for the preparation of a pharmaceutical formulation according to either of claims 3 and 4 wherein the carrier is sterile water.

7. A process for the preparation of a formulation according to claim 1 or 2 wherein said formulation is further adapted for oral administration.

8. A process for the preparation of a formulation according to claim 1, 2 or 7 wherein said formulation is made in the form of a tablet or capsule.

9. A process for the preparation of a formulation according to claim 1, 2 or 3 wherein said formulation is further adapted to provide sustained release of the active ingredient after oral administration.

10. A process for the preparation of a formulation according to claim 7 further comprising the addition of a flavouring agent.

11. A process for the preparation of a formulation according to any of claims 1 to 10 using between 10 and 1000 mg of the active ingredient.

12. A process for the preparation of a formulation according to any of claims 1 to 10 using between 20 and 700 mg of the active ingredient.

**Patentansprüche**

**Patentansprüche für folgende Vertragsstaaten : BE, CH, DE, FR, GB, IT, LI, LU, NL, SE**

1. Pharmazeutische Formulierung, umfassend ein pharmazeutisch annehmbares Derivat von 3'-Azido-3'desoxythymidin und einen pharmazeutisch annehmbaren Träger dafür, wobei die Formulierung eine Einheitsdosis zwischen 5 und 1500 mg des Wirkstoffes umfaßt.

2. Formulierung nach Anspruch 1, worin der Träger ein anderer als Wasser ist.

3. Formulierung nach Anspruch 1 oder 2, welche steril ist.

4. Formulierung nach Anspruch 3, die zur Verabreichung durch Injektion angepaßt ist.

5. Formulierung nach Anspruch 4, die in einer verschlossenen Ampulle enthalten ist.

6. Formulierung nach einem der Ansprüche 3 und 4, worin der Träger steriles Wasser ist.

7. Formulierung nach Anspruch 1 oder 2, die für die orale Verabreichung angepaßt ist.

8. Formulierung nach einem der Ansprüche 1, 2 oder 7 in Form einer Tablette oder Kapsel.

9. Formulierung nach einem der Ansprüche 1, 2 oder 3, welcher eine anhaltende Freisetzung des Wirkstoffes nach oraler Verabreichung zur Verfügung stellt.

10. Formulierung nach Anspruch 7, welche ferner einen Geschmacksstoff umfaßt.

11. Formulierung nach einem der Ansprüche 1 bis 10, worin die Einheitsdosis zwischen 10 und 1000 mg des Wirkstoffes ist.

12. Formulierung nach einem der Ansprüche 1 bis 10, worin die Einheitsdosis zwischen 20 und 700 mg des Wirkstoffes ist.

13. Verfahren zur Herstellung einer Formulierung nach einem der Ansprüche 1 bis 12, welches das Inkontaktbringen eines pharmazeutisch annehmbaren Derivats von 3'-Azido-3'desoxythymidin mit dem pharmazeutisch annehmbaren Träger umfaßt.

**Patentansprüche für folgenden Vertragsstaat : AT**

1. Verfahren zur Herstellung einer pharmazeutischen Formulierung, umfassend das Inkontaktbringen eines pharmazeutisch annehmbaren Derivats von 3'-Azido-3'desoxythymidin als Wirkstoff mit einem Träger dafür außer zur Bildung einer einfachen, nicht-sterilen wäßrigen Lösung des Wirkstoffes, worin der Wirkstoff in einer Einheitsdosis zwischen 5 und 1500 mg dargereicht wird.

2. Verfahren zur Herstellung einer Formulierung nach Anspruch 1, worin der Träger ein anderer als Wasser ist.

3. Verfahren zur Herstellung einer Formulierung nach Anspruch 1 oder 2, worin die Bestandteile steril sind.

4. Verfahren zur Herstellung einer Formulierung nach Anspruch 3, worin die Formulierung zur Verabreichung durch Injektion angepaßt ist.

5. Verfahren zur Herstellung einer Formulierung nach Anspruch 4, ferner umfassend die Aufbewahrung in einer verschlossenen Ampulle.

6. Verfahren zur Herstellung einer Formulierung nach einem der Ansprüche 3 und 4, worin der Träger steriles Wasser ist.

7. Verfahren zur Herstellung einer Formulierung nach Anspruch 1 oder 2, worin die Formulierung ferner für die orale Verabreichung angepaßt ist.

8. Verfahren zur Herstellung einer Formulierung nach den Ansprüchen 1, 2 oder 7, worin die Formulierung in Form einer Tablette oder Kapsel hergestellt wird.

9. Verfahren zur Herstellung einer Formulierung nach Anspruch 1, 2 oder 3, worin die Formulierung ferner angepaßt ist, um eine anhaltende Freisetzung des Wirkstoffes nach oraler Verabreichung zur Verfügung zu stellen.

10. Verfahren zur Herstellung einer Formulierung nach Anspruch 7, ferner umfassend die Zugabe eines Geschmacksstoffes.

11. Verfahren zur Herstellung einer Formulierung nach einem der Ansprüche 1 bis 10 unter Verwendung von 10 bis 1000 mg des Wirkstoffes.

12. Verfahren zur Herstellung einer Formulierung nach einem der Ansprüche 1 bis 10 unter Verwendung von 20 bis 700 mg des Wirkstoffes.

## Revendications

### Revendications pour les Etats contractants suivants : BE, CH, DE, FR, GB, IT, LI, LU, NL, SE

1. Préparation pharmaceutique comprenant un dérivé pharmaceutiquement acceptable de l'azido-3' désoxy-3' thymidine, et un support pharmaceutiquement acceptable pour celui-ci, ladite préparation comprenant une dose unitaire entre 5 et 1500 mg de l'ingrédient actif.

2. Préparation selon la revendication 1, dans laquelle le support est autre que de l'eau.

3. Préparation selon la revendication 1 ou 2, qui est stérile.

4. Préparation selon la revendication 3, adaptée pour une administration par injection.

5. Préparation selon la revendication 4, contenue dans un flacon scellé.

6. Préparation selon l'une des revendications 3 ou 4, dans laquelle le support est de l'eau stérile.

7. Préparation selon la revendication 1 ou 2, adaptée pour une administration par voie orale.

8. Préparation selon l'une quelconque des revendications 1, 2 ou 7, sous la forme d'un comprimé ou d'une capsule.

9. Préparation selon l'une quelconque des revendications 1, 2 ou 3, qui assure une libération progressive de l'ingrédient actif, après administration par voie orale.

10. Préparation selon la revendication 7 comprenant, en outre, un agent aromatisant.

11. Préparation selon l'une quelconque des revendications 1 à 10, dans laquelle la dose unitaire est entre 10 et 1000 mg de l'ingrédient actif.

12. Préparation selon l'une quelconque des revendications 1 à 10, dans laquelle la dose unitaire est entre 20 et 700 mg de l'ingrédient actif.

13. Procédé de fabrication d'une préparation selon l'une quelconque des revendications 1 à 12, comprenant le fait de mettre un dérivé pharmaceutiquement acceptable de l'azido-3' désoxy-3' thymidine en association avec ledit support pharmaceutiquement acceptable.

### Revendications pour l'Etat contractant suivant : AT

1. Procédé de fabrication d'une préparation pharmaceutique, comprenant le fait de mettre un dérivé pharmaceutiquement acceptable de l'azido-3' désoxy-3' thymidine comme ingrédient actif, en association avec un support pour celui-ci, autre que formant une simple solution aqueuse, non stérile, de l'ingrédient actif, dans lequel l'ingrédient actif est présenté en une dose unitaire entre 5 et 1500 mg.

2. Procédé de fabrication d'une préparation selon la revendication 1, dans lequel le support est autre que de l'eau.

3. Procédé de fabrication d'une préparation selon la revendication 1 ou 2, dans lequel les ingrédients sont stériles.

4. Procédé de fabrication d'une préparation selon la revendication 3, dans lequel ladite préparation est adaptée pour une administration par injection.

5. Procédé de fabrication d'une préparation selon la revendication 4 qui comprend, en outre, la conservation dans

un flacon scellé.

6. Procédé de fabrication d'une préparation pharmaceutique selon l'une des revendications 3 ou 4, dans lequel le support est de l'eau stérile.

7. Procédé de fabrication d'une préparation selon la revendication 1 ou 2, dans lequel ladite préparation est, en outre, adaptée pour une administration par voie orale.

8. Procédé de fabrication d'une préparation selon la revendication 1, 2 ou 7, dans lequel ladite préparation est faite sous la forme d'un comprimé ou d'une capsule.

9. Procédé de fabrication d'une préparation selon la revendication 1, 2 ou 3, dans lequel ladite préparation est, en outre, adaptée pour assurer une libération progressive de l'ingrédient actif, après administration par voie orale.

10. Procédé de fabrication d'une préparation selon la revendication 7 qui comprend, en outre, l'addition d'un agent aromatisant.

11. Procédé de fabrication d'une préparation selon l'une quelconque des revendications 1 à 10, utilisant entre 10 et 1000 mg de l'ingrédient actif.

12. Procédé de fabrication d'une préparation selon l'une quelconque des revendications 1 à 10, utilisant entre 20 et 700 mg de l'ingrédient actif.